(19) Europäisches Patentamt
European Patent Office
Office européen des brevets

(11) Numéro de publication: **0 003 455**
**B1**

(12) **FASCICULE DE BREVET EUROPEEN**

(45) Date de publication du fascicule du brevet: **17.04.85**

(51) Int. Cl.[4]: **C 07 J 41/00,** A 61 K 31/785

(21) Numéro de dépôt: **79400038.0**

(22) Date de dépôt: **23.01.79**

(54) **Amino-3-cardénolides, procédé pour leur préparation, et médicaments les contenant.**

(30) Priorité: **27.01.78 FR 7802391**

(43) Date de publication de la demande:
**08.08.79 Bulletin 79/16**

(45) Mention de la délivrance du brevet:
**17.04.85 Bulletin 85/16**

(84) Etats contractants désignés:
**BE CH DE FR GB IT LU NL SE**

(56) Documents cités:
**FR-A-2 085 722**
**FR-A-2 100 951**
**FR-A-2 111 705**

**BULLETIN DE LA SOCIETE CHIMIQUE DE FRANCE, février 1973, Paris MARIE HELENE BOUCHER, ROSE JACQUESY, no. 117 "Reactions du borocyanohydrure I-Amination reductrice régioselectives de cétones steroidiques" pages 750-753**

(73) Titulaire: **ETABLISSEMENTS NATIVELLE S.A.**
**27, rue de la Procession**
**F-75015 Paris (FR)**

(72) Inventeur: **Jarreau, François-Xavier**
**5, rue Louis Hervé**
**F-78000 Versailles (FR)**
Inventeur: **Koenig, Jean-Jacques**
**1, rue Rodin**
**F-91380 Chilly-Mazarin (FR)**

(74) Mandataire: **L'Helgoualch, Jean et al**
**OFFICE PICARD 134 Boulevard de Clichy**
**F-75018 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 003 455 B1

**Description**

La présente invention, réalisée dans les laboratories du CERES (Centre Européen de Recherche Scientifique), concerne de nouveaux dérivés aminés de cardénolides, un procédé pour leur préparation, et leur application en thérapeutique.

Il est bien connu que de nombreuses substances naturelles dérivées d'hétérosides cardiotoniques sont utilisées en thérapeutique pour le traitement des insuffisances cardiaques. L'activité cardiotonique des cardénolides-glycosides, en particulier, dépend notamment de la structure de la partie cardénolide et de la nature de la chaîne sucrée greffée en 3β. En raison de la faible marge thérapeutique de ces substances naturelles, et des inconvénients qui en résultent pour leur utilisation, il est apparu intéressant de préparer des composés de structure voisine possédant une activité cardiotonique satisfaisante associée à une faible toxicité.

On a proposé de greffer un substituant approprié en position 3 de la partie cardénolide, et de préférence en 3β. Par exemple, le brevets français 2.085.722, 2.100.951 et 2.111.705, décrivent des dérivés de cardénolides substitués en 3α et 3β par un groupe amino ou alkylamino. Ainsi, le brevet français 2.085.722 décrit des amino-3 cardénolides portant en position 3 un groupe $-NHR_9$ où $R_9$ est un atome d'hydrogène, ou un groupe acyle ou benzoyle; le brevet français 2.100.951 décrit des cardénolides et bufadiénolides substitués en position 3 par un groupe amino primaire, secondaire ou tertiaire, ou 2-oxo-3-oxazolidinyle, ou uréido; et le brevet français 2.111.705 décrit des N-alkylamino-3 cardénolides. Ces dérivés sont présentés comme possédant une activité cardiotonique. Toutefois, la préparation de tels composés, comportant un substituant rattaché par un atome d'azote à la position 3 du cardénolide, s'avère d'autant plus délicate et difficile que le substituant est complexe et comporte lui-même un ou plusieurs groupes fonctionnalisés. La préparation de tels composés doit alors s'effectuer en plusieurs étapes et il est nécessaire de préparer tout d'abord le dérivé de cardénolide substitué en 3 par un groupe $NH_2$, par exemple par action de l'hydroxylamine sur un oxo-3 cardénolide pour former une oxime que l'on réduit par l'hydrogène en présence d'un catalyseur métallique; on peut également faire agir une amine primaire pour former une base de Schiff que l'on soumet à une hydrogénation catalytique. Cet amino-3 cardénolide peut ensuite servir d'intermédiaire pour la préparation d'autres composés substitués sur l'azote.

Ces procédés sont difficile à mettre en oeuvre en raison du grand nombre d'étapes qu'ils requièrent, et de la fragilité de la partie cardénolide, en particulier du cycle lactonique greffé en 17 qu'il faut éviter de saturer en contrôlant avec précaution l'étape de la réduction.

La présente invention a pour objet de nouveaux dérivés de cardénolides substitués en 3 par un groupe du type amine fonctionnalisée, ainsi que leur application en thérapeutique, notamment pour le traitement des insuffisances cardiaques et des troubles du rythme.

L'invention a également pour objet un procédé de préparation d'amino-3 cardénolides en une seule étape, avec un bon rendement, en évitant les inconvénients ci-dessus. Plus particulièrement, le procédé conforme à l'invention permet d'obtenir des groupes aminés, aussi bien primaires que secondaires, tertiaires, éventuellement fonctionnalisés, en position 3 du cardénolide, en une seule étape à partir d'une oxo-3 génine, sans réduire le cycle lactonique du cardénolide.

L'invention concerne le produits nouveaux obtenus en greffant sur le cardénolide, en 3α ou 3β, un groupe aminé fonctionnalisé. Plus particulièrement, l'invention a pour objet les dérivés du type alkylamino-3 cardénolide représentés par la formule générale (I) suivante:

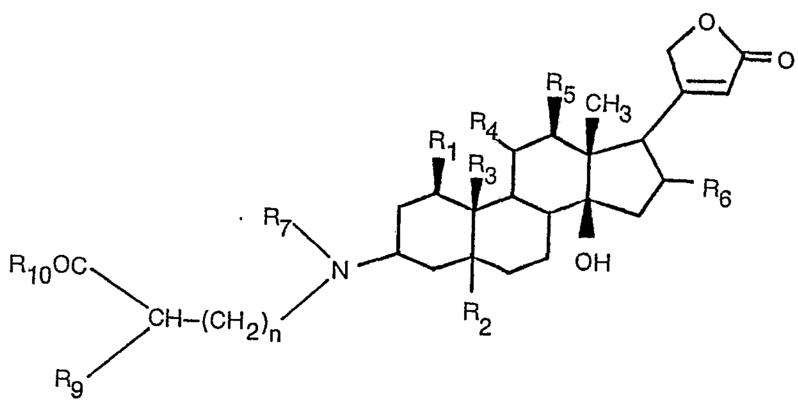

dans laquelle $R_1$, $R_2$, $R_4$ et $R_6$ représentent un atome d'hydrogène ou un groupe hydroxy; $R_3$ représente un groupe méthyle, hydroxyméthyle, ou formyl; $R_5$ représente un atome d'hydrogène, un groupe hydroxy ou un groupe acétoxy; $R_7$ représente un atome d'hydrogène, un groupe alkyle inférieur de 1 à 5 atomes de carbone, ou un groupe acyle; $R_9$ représente un groupe $-NR_{11}R_{12}$, $R_{11}$ et $R_{12}$, identiques ou différents, représentant un atome d'hydrogène, ou un groupe alkyle inférieur de 1 à 5 atomes de carbone, ou aryloxycarbonyle; $R_{10}$ représente un groupe hydroxy, alcoxy, aryloxy ou aralcoxycarbonyloxy; n est un entier de 0 à 6.

Conformément au procédé de l'invention on fait réagir une oxo-3 génine, telle que la digitoxigénone

ou la digoxigénone, avec un sel d'ammonium en présence d'un borocyanohydrure, dans un solvant organique tel qu'un alcool, pour former les dérivés de cardénolides représentés par la formule générale (I) ci-dessus.

Comme cardénolide de départ, on peut choisir des oxo-3 cardénolides que l'on obtient aisément à partir des génines correspondantes sous formes 3α ou 3β, suivant les méthodes classiques, et plus particulièrement la 3-digitoxigénone, la 3-digoxigénone, la oxo-3 acétoxy-12β digoxigénine, l'uzarigénone, etc.

Sur ces oxo-3 cardénolides, on fait agir un sel d'ammonium en 3α ou 3β, et par exemple un sel d'un acide monoaminé ou diaminé tel que la glycine, la taurine, l'alanine, la leucine, la lysine, l'ornithine ou l'acide diaminobutyrique, ou un sel d'ammonium d'un oligo-peptide. Il se forme dans ces conditions un intermédiaire immonium, au sein du milieu réactionnel, qui est réduit en amino-3 cardénolide en présence d'un borocyanohydrure de métal alcalin, suivant une réaction analogue à celle décrite par Borch et al. (JACS 1971, *93*, 2897) et Boutigue et al. (Bull. Soc. Chim. 1973 p. 750). La réaction s'effectue avantageusement en présence de borocyanohydrure de sodium ou de potassium, dans divers solvants tels que les alcools, et par exemple le méthanol ou l'isopropanol, ou l'eau et l'acétonitrile. Le procédé suivant l'invention se déroule sous pression normale, à une température comprise entre 0 et 40°C, et de préférence à la température ambiante.

Selon un mode de mise en oeuvre préférentiel du procédé suivant l'invention, on met en solution dans le méthanol ou l'isopropanol un oxo-3 cardénolide et un sel d'ammonium approprié, puis on ajoute du borocyanohydrure de sodium, à température ordinaire sous agitation. On maintient l'agitation pendant toute la durée de la réaction, puis on distille le solvant sous pression réduite. L'amino-3 cardénolide ainsi formé est alors extrait et purifié suivant les techniques usuelles.

Parmi les composés représentés par la formule (I) ci-dessus, l'invention concerne de préférence les composés pour lesquels $R_1$, $R_2$ et $R_6$ représentent un atome d'hydrogène ou un groupe hydroxy, $R_3$ représente un groupe alkyle inférieur, par exemple un groupe méthyle, ou un groupe hydroxyalkyle, par exemple un groupe hydroxyméthyle, ou un groupe formyle, $R_4$ représente un atome d'hydrogène, un groupe hydroxy ou un groupe acétoxy.

L'invention concerne tout particulièrement les dérivés d'amino-3 cardénolides et d'acides aminés, et de préférence les composés suivants, ainsi que leurs sels:

Composé A
la désoxy-3 ε-L-lysylamino-3α digitoxigénine.

Composé B
la désoxy-3 ε-L-lysylamino-3β digitoxigénine.

Composé C
la désoxy-3 N-(ε-L-lysyl) N'-acétylamino-3α digitoxigénine

Composé D
la désoxy-3 N-(ε-L-lysyl) N'-acétylamino-3β digitoxigénine.

Composé E
la désoxy-3 ε-L-lysylamino-3α acétoxy-12β digoxigénine.

Composé F
la désoxy-3 ε-L-lysylamino-3β acétoxy-12β digoxigénine.

Composé G
la désoxy-3 N-glycylamino-3α digitoxigénine.

Composé H
la désoxy-3 N-glycylamino-3β digitoxigénine.

Les composés de formule (I) conformes à la présente invention présentent l'avantage de comporter dans leur molécule à la fois un groupe acide $—COR_{10}$ et un groupe aminé basique, ce qui permet leur transformation sous forme de sels tant par action de bases que d'acides minéraux ou organiques.

L'invention s'étend également aux sels des dérivés d'amino-3 cardénolides, en particulier les sels pharmaceutiquement acceptables, par réaction avec des acides usuels tels que les acides chlorhydrique, sulfurique, phosphorique, acétique, propionique, oxalique, lactique, citrique, tartrique, ascorbique, aspartique, glutamique ou malonique, ou avec un hydroxyde alcalin, et par exemple l'hydroxyde de sodium, de potassium ou de lithium, un hydroxyde alcalino-terreux, tel que l'hydroxyde de magnésium ou de calcium. On peut également préparer des sels de métaux tels que l'aluminium, ou des sels d'ammonium.

Les sels peuvent être obtenus de façon usuelle, en faisant réagir en proportions sensiblement stoechiométriques le dérivé d'amino-3 cardénolide sous forme d'acide ou de base libre, avec un hydroxyde

ou un acide approprié, dans un solvant convenablement choisi en fonction de l'acide ou de la base, par exemple l'eau ou un alcool.

Les dérivés d'amino-3 cardénolides décrits ci-dessus sont obtenus suivant le procédé conforme à la présente invention sous forme de mélange de leurs isomères 3α et 3β. Ces isomères peuvent être séparés par les méthodes classiques, et par exemple par chromatographie ou cristallisation de leurs sels respectifs.

Les expérimentations pharmacologiques et toxicologiques effectuées sur les dérivés d'amino-3 cardénolides et leurs sels ont mis en évidence d'intéressantes propriétés permettant leur application en thérapeutique.

Les composés suivant l'invention présentent en effet une action tonicardiaque similaire à celle des hétérosides cardiotoniques connus, avec une meilleure marge thérapeutique c'est-à-dire un intervalle plus important entre la dose active et la dose toxique.

Ainsi on a constaté que les dérivés d'amino-3-cardénolides suivant l'invention présentent une activité inhibitrice de l'ATPase $Na^+$, $K^+$ dépendate, significative de l'activité des digitaliques, vérifiée sur le tissu de cerveau de rat.

La similitude entre l'action des composés de l'invention et celle des hétérosides cardiotoniques connus est confirmée par l'activité inotrope vérifiée sur le coeur de cobaye isolé et perfusé (Langendorff) et sur le coeur de chien in situ.

Les composés suivant l'invention se caractérisent en outre par une persistance de l'activité inhibitrice de l'ATPase membranaire, tandis que, au contraire, l'activité inotrope est variable en fonction de la nature de substituants greffés sur l'atome d'azote en position 3, et en fonction du cardénolide de départ lui-même. On constate par exemple que pour les composés précités, les pourcentages d'inhibition de l'ATPase membranaire dépendante du cerveau de cobaye, pour des doses de protéine enzymatique de 36 µg/mg et 0,36 µg/mg sont sensiblement équivalents à ceux de la digoxine et de la digitoxine.

Ces propriétés pharmacologiques comparables à celles des hétérosides cardiotoniques connus, malgré la présence d'un reste peptidique en position 3 du noyau stéroïdique, à la place des sucres des hétérosides, montrent que les dérivés d'amino-3-cardénolides suivant l'invention peuvent être utilisés pour le traitement des affections cardiaques, et plus particulièrement l'insuffisance cardiaque et les troubles du rythme.

Les nouveaux composés suivant l'invention peuvent être administrés sous les formes usuelles contenant une quantité pharmacologiquement efficace du composé en tant que principe actif dilué dans les supports pharmaceutiquement utilisables, par example sous forme de comprimés, gélules, capsules, dragées, suppositoires, solutés injectables ou sirops.

Comme diluant solide pour la préparation des comprimés, on peut utiliser le lactose, le mannitol, le sorbitol, l'amidon, la polyvinylpyrrolidone, le stéarate de magnésium ou d'aluminium, la poudre de cellulose, la silice colloidale, le talc, etc.

Des solutions injectables peuvent être préparées au moyen de diluants tels que l'eau bi-distillée, le propylèneglycol, une solution hydroalcoolique, ou un mélange de ces diluants, de préférence en présence d'un conservateur approprié choisi parmi ceux utilisés communément dans la technique.

Des formes buvables peuvent également être préparées, par exemple des solutés contenant le composé de l'invention dissous dans de l'eau et du glycérol en présence d'un édulcorant et d'un antioxydant, ou des suspensions de composé de l'invention dans une solution aqueuse de saccharose en présence d'un épaississant, d'un édulcorant, et d'un anti-oxydant.

Toutes les formulations adaptées aux divers modes d'administration, c'est-à-dire par voie orale, parentérale ou rectale, peuvent être utilisées, le médicament étant associé en tant que principe actif aux excipients pharmaceutiquement acceptables convenablement choisis.

A titre d'exemple on peut citer les formulations suivantes:

Comprimés:

| | |
|---|---|
| A—Composé B | 0,25 mg |
| Lactose | 134,75 |
| Talc | 15,0 |
| | 150,0 mg |
| B—Composé F | 0,25 mg |
| Amidon | 81,25 |
| Silice colloidale | 0,50 |
| Cellulose microcristalline | 18,00 |
| | 100 mg |

Soluté injectable:

| | |
|---|---|
| Composé B | 0,01 mg |
| Conservateur | 0,001 |
| Eau q.s.p. | 1 ml |

Suspension buvable:

| | |
|---|---|
| Composé F | 0,25 g |
| Epaississant | 10 g |
| Saccharose | 25 g |
| Edulcorant | 1 g |
| Antioxydant | 0,0001 g |
| Eau q.s.p. | 100 ml |

La posologie peut varier selon le sujet traité et l'affection en cause, les doses administrées journellement étant généralement comprises entre 0,01 et 1 mg pour l'administration orale chez l'homme.

Les exemples de préparation décrits ci-après illustrent l'invention sans en limiter la portée.

Exemple 1

Désoxy-3 N-ω-(benzyloxy glycinate)-amino-3 digitoxigénine. (Isomères 3α et 3β).

On laisse 30 minutes sous agitation à température ambiante, 320 mg de digitoxigénone, et 625 mg de tosylate de 0-benzyl glycine, dans 10 ml de propanol-2. On ajoute 70 mg de borocyanohydrure de sodium.

Après 4 jours d'agitation à température ambiante, on distille à sec, et on reprend le résidue par de l'eau. On alcalinise par du bicarbonate de sodium, on extrait par de l'acétate d'éthyle pour obtenir après distillation 601 mg de résidu. On dissout celui-ci dans 10 ml de THF bouillant, et on lui ajoute 230 mg d'acide oxalique dans le THF bouillant. Après cristallisation à froid, filtration des cristaux, que l'on reprend par de l'ammoniaque dilué, on extrait par de l'acétate d'éthyle. Les extraits, lavés, séchés et distillés à sec, fournissent 143 mg de l'isomère 3β sous forme d'huile (Rdt=32%).

Les eaux mères distillées à sec sont reprises par de l'ammoniaque dilué et extraites par de l'acétate d'éthyle. Les extraits lavés, séchés, et distillés à sec, fournissent 293 mg de l'isomère 3α impur, sous forme d'huile (Rdt.=65%.

Isomère 3α $C_{32}H_{43}O_5N$ M=521,66

IR (film) ν=3450, 3020, 1780, 1740, 1660, 1620, 1500, 1170, 1028 $cm^{-1}$

CCM ($CH_2Cl_2$-MeOH, 90-10) Rf=0,75

Isomère 3β

IR (film) ν=3460, 3340, 3020, 1785, 1745, 1670, 1622, 1500, 1180, 1028 $cm^{-1}$

RMN ($CDCl_3$) δ=0,88 et 0,93 (2s, $CH_3$), 2,85 (1H) 3,0 (1H), 3,55 (2H), 5,1 (2H) 5,33 (2H), 6,08 (1H), 7,6 (5H) ppm.

CCM ($CH_2Cl_2$-MeOH, 90-10) Rf.=0,80

Exemple 2

Désoxy-3 N-glycyl amino-3α digitoxigénine.

On hydrogénolyse 150 mg de l'isomère 3α obtenu dans l'exemple 8 en solution dans 15 ml de méthanol, en présence de 30 mg de palladium à 5% sur carbonate de calcium, pendant 20 h. On filtre et distille le filtrat à sec. Après purification du résidu (115 mg) par lavage dans l'acétate d'éthyle et trituration dans l'éther, 90 mg de désoxy-3 N-glycylamino-3α digitoxigénine sont ainsi obtenus. composé G (Rdt=75%).

$C_{25}H_{35}O_5N$ M=429,53

F (Kofler)=195—210°C (déc.)

CCM ($CHCl_3$-EtOH-$NH_4OH$, 50-45-15) Rf=0,51

En opérant de la même façon à partir de 110 mg de l'isomère β obtenu dans l'exemple 1, on prépare 88 mg de cristaux de désoxy-3 N-glycylamino-3β digitoxigénine composé H (Rdt=76%).

F (Kofler): 225—230°C (déc)

IR (Nujol) ν=3530, 3400, 1788, 1758, 1720, 1625, 1598 et 1034 $cm^{-1}$

RMN ($CDCl_3+CD_3OD$) δ=0,88 et 1,03 (2s, $CH_3$), 2,83 (1H) 3,36 (2H) 3,1—3,7 (1H), 4,98 (2H) 5,91 (1H) ppm.

CCM ($CHCl_3$-EtOH-$NH_4OH$, 50-45-15) Rf=0,53

Exemple 3

Désoxy-3 ε-(α-N-benzyloxycarbonyl O-benzyl)-(L)-lysylamino-3 digitoxigénine.

A 1,48 g de 3-digitoxigénone et 3,43 g de benzènesulfonate de α-N-benzyloxycarbonyl O-benzyl-(L)-lysine en solution dans 50 ml de méthanol, on ajoute sous agitation et à température ordinaire 0,32 g de borocyanohydrure de sodium. Après agitation pendant 6 heures, on distille le méthanol à sec sous pression réduite, et on reprend le résidue par 100 ml d'une solution saturée de bicarbonate de sodium. On extrait par du chlorure de méthylène. On lave, sèche et distille à sec puis on reprend le résidue (4,15 g) par 100 ml d'acétate d'éthyle. Après lavage avec de l'acide chlorhydrique N, de l'eau une solution saturée de bicarbonate de sodium, et enfin de l'eau, on réextrait de la même manière avec de l'acétate d'éthyle. Les fractions organiques sont séchées et distillées à sec et on obtient un résidu (3,27 g) constitué d'un mélange des isomères 3α et 3β contenant encore de l'acide aminé. On le dissout dans 10 ml de méthanol et l'ajoute goutte à goutte à 0,54 g d'acide oxalique en solution dans 10 ml de méthanol à reflux. Le résidu peut être cristallisé dans de l'acétate d'éthyle contenant un peu d'éther. On recristallise le premier jet (1,07 g constitué de l'oxalate du produit le moins polaire) dans l'acétate d'éthyle et on libère la base par dissolution dans l'eau, alcalinisation et extraction par du chloroforme. Après sèchage et évaporation, on obtient 0,88 g du produit le moins polaire qui est l'isomère 3β de la désoxy-3[ε-(α-N-benzyloxycarbonyl-O-benzyl-L-lysyl]-amino-3 digitoxigénine (Rdt=30%).

On évapore à sec les eaux mères du deuxième jet, on lave le résidu (2,3 g constitué essentiellement de l'oxalate du produit le plus polaire) avec de l'éther et on libère la base. Après lavage au toluène il reste 1,52 g d'une huile qui est l'isomère 3α de la désoxy-3[ε-(α-N-benzyloxycarbonyl, O-benzyl-L-lysyl]amino-3-digitoxigénine (Rdt: 53%).

Isomère 3β

F (Kofler) (Oxalate)=191—192°C

Spectre IR (Nujol): 3450, 3340, 1780, 1745, 1725, 1620, 1530 $cm^{-1}$

Spectre de RMN ($CDCl_3$) δ=0,85 et 0,95 (2s, $CH_3$), 2,60 (3H) 2,99 (s, 1H), 4,35 (1H), 4 à 4,7 (NH et OH), 4,85 (2H) 5,05 et 5,10 (2s, $CH_2$), 5,52 (NH, d, J=7) 5,80 (s, 1H) 7,30 (s, 10H) ppm.

Isomère 3α

IR (Nujol): 3440, 3340, 1780, 1740, 1715, 1620, 1530 $cm^{-1}$

CCM: $CH_2Cl_2$-MeOH 99-1 atmosphère $NH_3$

Isomère 3α Rf: 0,48

Isomère 3β Rf: 0,54

Exemple 4

Désoxy-3 N-ε-(α-N-benzyloxycarbonyl O-benzyl)-(L)-lysyl N'-acétylamino-3 digitoxigénine.

a) Isomère 3β

On place 400 mg de l'isomère 3β obtenu comme indiqué dans l'exemple 3 en solution dans 2,5 ml d'anhydride acétique et 5 ml de pyridine et on laisse réagir 3 h. à température ambiante. A la fin de la réaction, on jette sur de l'eau glacée, acidifie à pH 2—3 par de l'acide chlorhydrique et extrait par du chlorure de méthylène les fractions organiques sont lavées par du bicarbonate de sodium, puis par de l'eau, séchées et distillées à sec. Le résidu est constitué de 420 mg de désoxy-3 N-ε (α-N-benzyloxycarbonyl O-benzyl)-(L)-lysyl N'-acétylamino-3β digitoxigénine (Rdt: 98%).

Spectre IR (Nujol): 3430, 3340, 1755, 1745, 1720, 1620 et 1530 $cm^{-1}$

CCM ($CH_2Cl_2$-MeOH 95-5) Rf=0,28

b) Isomère 3α

On opère comme précédemment à partir de 760 mg de l'isomère 3α de l'exemple 3, et après extraction on chromatographie le résidu (779 mg) sur 40 g de silice Merck 40. Les fractions éluées par le mélange chlorure de méthylène-méthanol (98-2) fournissent 400 mg de désoxy-3 N-ε-(α-N-benzyloxycarbonyl O-benzyl)-(L)-lysyl N'-acétylamino-3α digitoxigénine (Rdt: 50%).

CCM: ($CH_2Cl_2$-MeOH 95-5) Rf=0,27

Exemple 5

Désoxy-3 ε-(L)-lysylamino-3 digitoxigénine.

a) Isomère 3α

On dissout 850 mg de l'isomère 3α de l'exemple 3 dans 85 ml de méthanol et on hydrogénolyse en présence de 210 mg de palladium à 5% sur carbonate de calcium pendant 6 h. Après filtration et distillation à sec, le résidu (662 mg) est lavé par du chloroforme, puis trituré dans un mélange d'acétate d'éthyle et d'éther isopropylique. Cela permet d'obtenir 323 mg d'un insoluble cristallin jaune constitué de désoxy-3 ε-(L)-lysylamino-3α digitoxigénine.

Composé A (Rdt: 47%).

F (Kofler) 190°—200°C déc.

Spectre IR (Nujol): 3400, 1780, 1755, 1745, 1720, 1620 $cm^{-1}$

Spectre de RMN ($CD_3OH$): δ=0,87 et 0,95 (2s, $CH_3$) 2,97 (3H), 3,37 (1H) 4,05 (1H) 5,85 (s, 1H) ppm.
CCM ($CHCl_s$-EtOH-$NH_4OH$, 50-45-15) Rf=0,17

b) Isomère 3β

On dissout 439 mg de l'isomère 3β obtenu dans l'exemple 3 dans 40 ml de méthanol et hydrogénolyse en présence de 110 mg de palladium à 5% sur carbonate de calcium pendant 3h30. Après filtration et distillation du solvant à sec, on cristallise le résidu (336 mg) dans du propanol-2. Cela fournit 252 mg de cristaux de désoxy-3ε-(L)-lysylamino-3β digitoxigénine. Composé B (Rdt: 83%).

F (Kofler) 185°—195°C déc.
IR (Nujol): 3400, 1780, 1760, 1740, 1725, 1620 $cm^{-1}$
RMN ($CD_3OD$): δ=0,88 et 1,00 (2s, $CH_3$), 2,8 (3H), 5,85 (s, 1H) ppm.
CCM ($CHCl_3$-EtOH-$NH_4OH$ 50-45-15) Rf=0,28

Exemple 6

Désoxy-3 N-(ε-L-lysyl) N'-acétylamino-3 digitoxigénine

a) Isomère 3α

On hydrogénolyse 385 mg de l'isomère 3α obtenu dans l'exemple 4 dans 40 ml de méthanol en présence de 97 mg de palladium à 5%. sur carbonate de calcium pendant 2 h.30. On filtre, distille à sec, et cristallise le résidu (282 mg) dans le mélange propanol-2/acétate d'éthyle. Cela permet d'obtenir 212 mg de cristaux de désoxy-3 N-(ε-(L)-lysyl) N'-acétylamino-3α digitoxigénine. Composé C. (Rdt: 78%).

F (Kofler) 195—205°C déc.
Spectre IR (Nujol): 3420, 1780, 1755, 1745, 1720 et 1615 $cm^{-1}$
CCM ($CHCl_3$-EtOH-$NH_4OH$- 50-45-15) Rf=0,42

b) Isomère 3β

On effectue l'hydrogénolyse de 420 mg de l'isomère 3β obtenu dans l'exemple 4, dans 50 ml de méthanol, en présence de 100 mg de palladium à 5% sur carbonate de calcium, pendant 3 h. On filtre, distille à sec et cristallise le résidu (304 mg) dans le mélange méthanol-acétate d'éthyle. Cela permet d'obtenir 211 mg de cristaux blancs de désoxy-3 N-(ε-(L)-lysyl), N'-acétylamino-3β digitoxigénine. Composé D (Rdt: 74%).

F (Kofler) 198—205°C déc.
Spectre IR (Nujol): 3460, 3280, 3040, 1800, 1755, 1745, 1735, 1720 et 1615 $cm^{-1}$
CCM ($CHCl_3$-EtOH-$NH_4OH$- 50-45-15) Rf=0,52

Exemple 7

Désoxy-3 ε-(α-N-benzyloxycarbonyl O-benzyl)-L-lysylamino-3 acétoxy-12β digoxigénine.

On dissout 1,5 g de oxo-3 acétoxy-12β digoxigénine et 2,92 g de benzènesulfonate de α-N-benzyloxycarbonyl O-benzyl-L-lysine, en solution dans 100 ml de propanol-2. Après 30 minutes sous agitation à température ordinaire, on ajoute 0,22 g de borocyanohydrure de sodium. Après une nuit de réaction on distille la majeure partie du solvant, et noie dans 100 ml d'acide chlorhydrique N. Après extraction de la fraction acide par de l'acétate d'éthyle, lavage des fractions organiques par de l'acide chlorhydrique puis par de l'eau, on sèche et distille à sec l'acétate d'éthyle. Le résidu (1,65 g) est constitué d'acide aminé, de traces d'amines et de produit de départ. On alcalinise à pH 8—9 les fractions acides par de l'ammoniaque concentré et extrait par du chlorure de méthylène. Après lavage, sèchage et distillation à sec de l'extrait organique, le résidue (2,16 g) est constitué de désoxy-3 ε-(α-N-benzyloxycarbonyl O-benzyl)-L-lysylamino-3β acétoxy-12β digoxigénine (Rdt: 73%).

Ce résidu est dissous dans 10 ml de méthanol que l'on ajoute goutte à goutte à une solution à reflux de 0,31 g d'acide oxalique un solution dans 10 ml de méthanol à reflux. On distille à sec et cristallise le résidu dans du propanol-2, puis on le triture dans l'éther.

Les cristaux obtenus (479 mg) fournissent, après dissolution dans l'eau, alcalisation par l'ammoniaque, extraction par le chloroforme, sèchage et distillation à sec, de l'extrait, 422 mg du produit le moins polaire qui est la désoxy-3ε-(α-N-benzyloxycarbonyl O-benzyl)-L-lysylamino-3β acétoxy-12β digoxigénine (Rdt: 16%)

Isomère 3β

F (Kofler) (oxalate)=186—194°C déc.
Spectre IR (Nujol): 3430, 3250, 1780, 1755, 1730, 1635 et 1540 $cm^{-1}$
CCM $CH_2Cl_2$-MeOH (99-1 atmosphère $NH_3$) Rf=0,50

On distille à sec les eaux mères du troisième jet, et on libère la base par alcalinisation en suspension dans l'ammoniaque dilué et extraction au chloroforme. Le résidu est trituré dans de l'éther isopropylique. L'insoluble solide obtenu (720 mg) est constitué de désoxy-3-ε(α-N-benzyloxycarbonyl O-benzyl)-L-lysylamino-3β acétoxy-12β digoxigénine. (Rdt: 25%).

Isomère 3α

IR (Nujol): 3420, 3300, 1780, 1750, 1735, 1620 et 1525 $cm^{-1}$
CCM $CH_2Cl_2$-MeOH (99-1 atmosphère $NH_3$) Rf: 0,46

Exemple 8

Désoxy-3 ε-L-lysylamino-3 acétoxy-12β digoxigénine.

a) Isomère 3α

L'hydrogénolyse de 580 mg de l'isomère 3α obtenu dans l'exemple 7 dans 70 ml de méthanol en présence de 145 mg de palladium à 5% sur carbonate de calcium pendant 6 h. permet d'obtenir, après filtration, distillation à sec et cristallisation du résidu (425 mg) dans un mélange méthanol-acétate d'éthyle, 195 mg de cristaux de désoxy-3 ε-L-lysylamino-3α acétoxy-12β digoxigénine. Composé E. (Rdt: 46%).

F (Kofler): 185—195°C déc.

Spectre IR (Nujol): 3400, 1780, 1755, 1745 et 1620 $cm^{-1}$

Spectre de RMN ($CD_3OD$): δ=0,87 0,94 et 2,08 (3s, $CH_3$) 2,88 (3H) 3,6 (1H) 4,15 (1H) 5,85 (s, 1H) ppm.

CCM $CHCl_3$-EtOH-$NH_4OH$, 50-45-15 Rf=0,15

b) Isomère 3β

L'hydrogénolyse de 325 mg de l'isomère 3β obtenu dans l'exemple 7, dans 40 ml de méthanol en présence de 80 mg de palladium à 5% sur carbonate de calcium pendant 4h. permet d'obtenir, après filtration, distillation à sec et cristallisation du résidu (238 mg) par lavage par du propanol-2 et trituration dans l'éther, 154 mg de cristaux de désoxy-3 ε-L-lysylamino-3β acétoxy-12β digoxigénine. Composé F (Rdt: 66%).

F (Kofler) 180—195°C déc.

Spectre IR (Nujol): 3400, 1780, 1755, 1740 et 1620 $cm^{-1}$

Spectre de RMN ($CD_3OD$): δ=0,86 0,98 et 2,10 (3s, $CH_3$) 2,82 (3H) 5,85 (s, 1H) ppm.

CCM $CHCl_3$-EtOH-$NH_4OH$- 50-45-15 Rf: 0,25

**Revendications**

1. Dérivés d'alkylamino-3 cardénolides représentés par la formule générale (I):

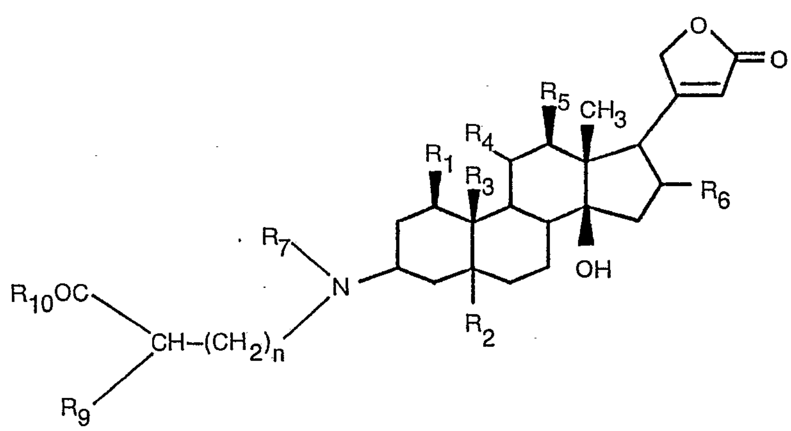

dans laquelle $R_1$, $R_2$, $R_4$ et $R_6$ représentent un atome d'hydrogène ou un groupe hydroxy; $R_3$ représente un groupe méthyle, hydroxyméthyle, ou formyle; $R_5$ représente un atome d'hydrogène, un groupe hydroxy ou un groupe acétoxy; $R_7$ représente un atome d'hydrogène, un groupe alkyle inférieur de 1 à 5 atomes de carbone, ou un groupe acyle; $R_9$ représente un groupe —$NR_{11}R_{12}$, $R_{11}$ et $R_{12}$, identiques ou différents, représentant un atome d'hydrogène, ou un groupe alkyle inférieur de 1 à 5 atomes de carbone ou aryloxycarbonyl; $R_{10}$ représente un groupe hydroxy, alcoxy, aryloxy ou aralcoxycarbonyloxy; n est un entier de 0 à 6; ainsi que leurs sels de bases ou d'acides minéraux et organiques.

2. Dérivés d'alkylamino-3 cardénolides selon la revendication 1, caractérisés en ce qu'il sont choisis parmi le groupe constitué par la désoxy-3 ε-L-lysylamino-3α digitoxigénine, la désoxy-3 ε-L-lysylamino-3β digitoxigénine, la désoxy-3 N-(ε-L-lysyl) N'-acétylamino-3α digitoxigénine, et la désoxy-3 N-(ε-L-lysyl) N'-acétylamino-3β digitoxigénine.

3. Dérivés d'alkylamino-3 cardénolides selon la revendication 1, caractérisés en ce qu'ils sont choisis parmi le groupe constitué par la désoxy-3 N-glycylamino-3α digitoxigénine, et la désoxy-3 N-glycylamino-3β digitoxigénine.

4. Dérivés d'alkylamino-3 cardénolides selon la revendication 1, caractérisés en ce qu'ils sont choisis parmi le groupe constitué par la désoxy-3 ε-L-lysylamino-3α acétoxy-12β digoxigénine et la désoxy-3 ε-L-lysylamino-3β acétoxy-12β digoxigénine.

5. Procédé de préparations de dérivés d'alkylamino-3 cardénolides selon la revendication 1, caractérisé en ce que l'on fait réagir une oxo-3 génine avec un sel d'ammonium en présence d'un borocyanohydrure dans un solvant organique.

6. Procédé selon la revendication 5, caractérisé en ce que le borocyanohydrure est ajouté à l'oxo-3 génine et au sel d'ammonium en solution dans un solvant qui est distillé après réaction.

7. Procédé selon l'une quelconque des revendications 5 et 6, caractérisé en ce que le sel d'ammonium est chois parmi un sel d'un acide monoaminé ou diaminé, ou un sel d'ammonium d'un oligopeptide.

8. Procédé selon l'une quelconque des revendications 5 et 6, caractérisé en ce qu'on utilise un borocyanohydrure de métal alcalin.

9. Procédé selon l'une quelconque des revendications 5 à 8, caractérisé en ce que la réaction est effectuée à température ambiante, sous agitation.

10. Compositions pharmaceutiques caractérisées en ce qu'elles contiennent, à titre de principe actif, un dérivé de cardénolide selon l'une quelconque des revendications 1 à 4, associé à un ou plusieurs excipients pharmaceutiquement acceptables, et, le cas échéant, à un ou plusieurs autres principes actifs.

**Patentansprüche**

1. 3-Alkylamino-cardenolidderivate der allgemeinen Formel (I)

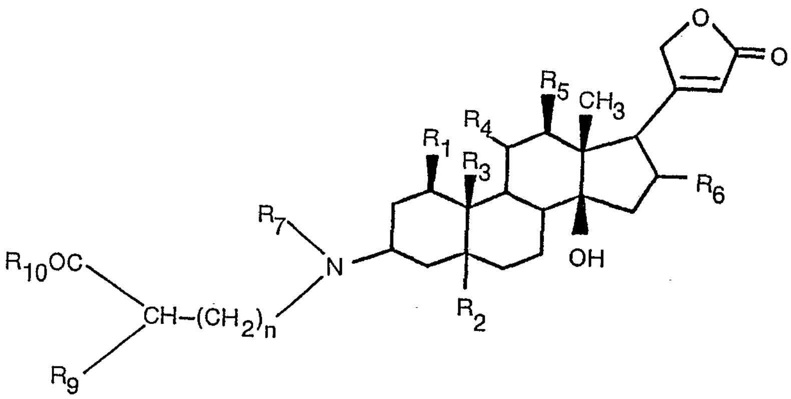

in der $R_1$, $R_2$, $R_4$ und $R_6$ ein Wasserstoffatom oder eine Hydroxygruppe; $R_3$ eine Methylgruppe, eine Hydroxymethylgruppe oder eine Formylgruppe; $R_5$ ein Wasserstoffatom, eine Hydroxygruppe oder eine Acetoxygruppe; $R_7$ ein Wasserstoffatom, eine niedere Alkylgruppe mit 1 bis 5 Kohlenstoffatomen, oder eine Acylgruppe, $R_9$ ein $-NR_{11}R_{12}$ Gruppe, worin $R_{11}$ und $R_{12}$, gleichartig oder verschieden, ein Wasserstoffatom oder eine Alkylgruppe mit 1 bis 5 Kohlenstoffatomen, oder eine Aryloxycarbonylgruppe darstellen; $R_{10}$ eine Hydroxygruppe, eine Alkoxygruppe, eine Aryloxygruppe, oder eine Aralkoxycarbonyloxygruppe bedeuten; n is von 0 bis 6; sowie die anorganischen oder organischen Salze dieser Derivate.

2. 3-Alkylamino-cardenolidderivate nach Anspruch 1, dadurchgekennzeichnet, dass sie aus Desoxy-3 ε-L-Lysylamino-3-α Digitoxigenin, Desoxy-3 ε-L-Lysylamino-3β Digitoxigenin, Desoxy-3 N-(ε-L-Lysyl) N'-Acetylamino-3α Digitoxigenin, und Desoxy-3 (N-(ε-L-Lysyl) N'-Acetylamino-3β Digitoxigenin bestehen.

3. 3-Alkylaminocardenolidderivate nach Anspruch 1, dadurch gekennzeichnet, dass sie aus Desoxy-3 N-Glycylamino-3α Digitoxigenin und Desoxy-3 N-Glycylamino-3β Digitoxigenin bestehen.

4. 3-Alkylaminocardenolidderivate nach Anspruch 1, dadurch gekennzeichnet, dass sie aus Desoxy-3 ε-L-Lysylamino-3α Acetoxy-12β Digoxigenin und Desoxy-3 ε-L-Lysylamino-3β Acetoxy-12β Digoxigenin bestehen.

5. Verfahren zur Herstellung der 3-Alkylaminocardenolidderivate nach Anspruch 1, dadurchgekennzeichnet, dass man eine Reaktion zwischen einer 3-Oxo-Genin und einem Ammonium Salz in Gegenwart von Borocyanohydrid in einem organischen Lösungsmittel überführt.

6. Verfahren nach Anspruch 5, dadurch gekennzeichnet, dass das Borocyanohydrid mit der 3-Oxo-Genin und dem Ammonium Salz in einem nach der Reaktion destillierten Lösungsmittel versetz wird.

7. Verfahren nach einem der Ansprüche 5 und 6, dadurch gekennzeichnet, dass das Ammonium Salz aus Monoamino oder Diamino Saüre besteht.

8. Verfahren nach einem der Ansprüche 5 und 6, dadurch gekennzeichnet, dass main ein Borocyanohydrid von Alkalimetal verwendet.

9. Verfahren nach Anspruch 8, dadurch gekennzeichnet, dass man die Reaktion bei Raumtemperatur unter Rühren ablaufen lässt.

10. Pharmazeutische Zubereitungen, dadurch gekennzeichnet, dass sie als Wirkstoff ein Cardenolidderivate nach einem der Ansprüche 1 bis 4, sowie ein oder mehrere pharmazeutisch verträglichen Trägermateriale und/oder andere Wirkstoffe enthalten.

## Claims

1. 3-alkylamino-cardenolide derivatives represented by the general formula (I)

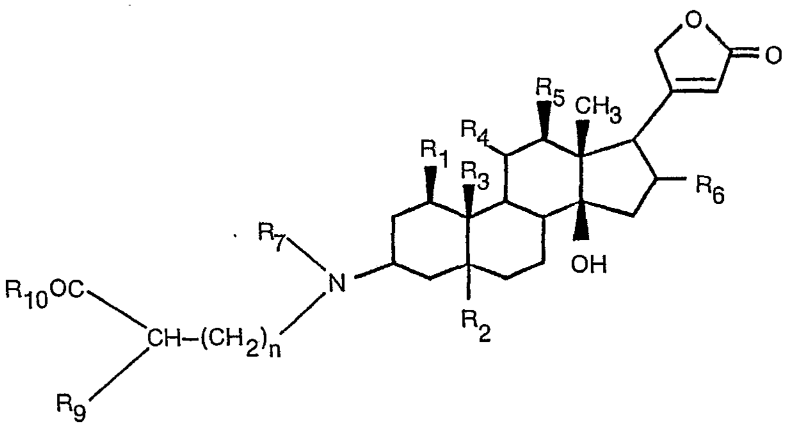

wherein $R_1$, $R_2$, $R_4$ and $R_6$ represent a hydrogen atom or a hydroxy group; $R_3$ represents a methy, hydroxymethyl or formyl group; $R_5$ represents a hydrogen atom, a hydroxy or acetoxy group; $R_7$ represents a hydrogen atom, a lower alkyl group from 1 to 5 carbon atoms or an acyl group; $R_9$ represents a $-NR_{11}R_{12}$ group, $R_{11}$ and $R_{12}$, which may be the same or different, representing a hydrogen atom or a lower alkyl group from 1 to 5 carbon atoms, or an aryloxycarbonyl group; $R_{10}$ represents a hydroxy, alkoxy, acyloxy or aralkoxycarbonyloxy group; n is an integer from 0 to 6; and mineral and organic basic or acid salts thereof.

2. 3-alkylamino-cardenolide derivatives according to claim 1, characterized in that they are selected from the group consisting of deoxy-3 ε-L-lysylamino-3α digitoxigenin, deoxy-3 ε-L-lysylamino-3β digitoxigenin, deoxy-3 N-(ε-L-lysyl) N′-acetylamino-3α digitoxigenin, and deoxy-3 N-(ε-L-lysyl) N′-acetylamino-3β digitoxigenin.

3. 3-alkylamino-cardenolide derivatives according to claim 1, characterized in that they are selected from the group consisting of deoxy-3 N-glycylamino-3α digitoxigenin and deoxy-3 N-glycylamino-3β digitoxigenin.

4. 3-alkylamino-cardenolide derivatives according to claim 1, characterized in that they are selected from the group consisting of deoxy-3 ε-L-lysylamino-3α acetoxy-12β digoxigenin, and deoxy-3 ε-L-lysylamino-3β acetoxy-12β digoxigenin.

5. A process for preparation of 3-alkylamino cardenolide derivatives according to claim 1, characterized in that it comprises reacting a 3-oxo-genin with an ammonium salt in the presence of a borocyanohydride in an organic solvent.

6. A process according to claim 5, characterized in that the borocyanohydride is added to the 3-oxo-genin and the ammonium salt dissolved in a solvent which is distilled after reaction.

7. A process according to any one of claims 5 and 6, characterized in that the ammonium salt is selected from a diamino- or monoamino-acid salt, or an oligopeptide ammonium salt.

8. A process according to any one of claims 5 and 6, characterized in that an alkali metal borocyanohydride is used.

9. The process according to any one of claims 5 to 8, wherein the reaction is carried out at room temperature, under stirring.

10. Pharmaceutical compositions characterized in that they comprise, as an active ingredient, a cardenolide derivative according to any of claims 1 to 4 with one or more pharmaceutically acceptable carriers, and possibly, one or more other active principles.